# EUROPÄISCHE PATENTSCHRIFT

(11) **EP 3 116 845 B1**
(45) Veröffentlichungstag und Bekanntmachung des Hinweises auf die Patenterteilung: **18.07.2018**
(21) Anmeldenummer: 15709452.5
(22) Anmeldetag: 05.03.2015
(51) Int. Cl.: C07C 29/141, C07C 29/90, C07C 31/20

(54) **VERFAHREN ZUM ABBAU VON FORMIATEN**
METHOD FOR BREAKDOWN OF FORMATES
PROCÉDÉ POUR LA DÉGRADATION DE FORMIATES

(30) Priorität: 12.03.2014 EP 14159198
(43) Veröffentlichungstag der Anmeldung: 18.01.2017
(73) Patentinhaber: BASF SE, 67056 Ludwigshafen am Rhein (DE)
(72) Erfinder: PINKOS, Rolf, 67098 Bad Dürkheim (DE); MARION, Nicolas, 68161 Mannheim (DE); KRONEMAYER, Helmut, 69221 Dossenheim (DE)
(86) Internationale Anmeldenummer: PCT/EP2015/054619
(87) Internationale Veröffentlichungsnummer: WO 2015/135830

(56) Entgegenhaltungen:
- WO-A1-2004/092097
- WO-A1-2007/006719
- CN-A- 103 449 970

## Beschreibung

Die Erfindung betrifft ein Verfahren zum Abbau von Formiaten mittels eines Lanthan-haltigen Katalysators in formiathaltigen Stoffgemischen und die Verwendung des Katalysators zu dem vorgenannten Zweck.

Im Sinne der Erfindung sind unter formiathaltigen Stoffgemischen insbesondere Stoffgemische enthaltend Carbonylverbindungen zu verstehen, die beispielsweise durch eine Aldolreaktion von Alkanonen oder Alkanalen mit Formaldehyd gebildet wurden, wie z.B. Methylolalkanale, sowie deren entsprechenden Hydrierprodukte. Diese Produkte enthalten neben den vorgenannten Carbonylverbindungen auch Formiate.

Die Herstellung von Methylolalkanalen und ein Verfahren zu deren Hydrierung mit Hilfe eines CuO/Al₂O₃-Katalysators werden in der WO 2004/092097 A1 beschrieben.

WO 2011/141470 A1 beschreibt die Umsetzung von Isobutyraldehyd mit wässrigem Formaldehyd in Gegenwart von Trimethylamin zu Hydroxypivalinaldehyd (HPA). Der HPAenthaltende Reaktionsaustrag wird mit einem Hydrierkatalysator zu Neopentylglykol (NPG) hydriert. Lanthan-haltige Katalysatoren werden nicht offenbart.

WO 2007/006719 beschreibt ein weiteres Verfahren zur Hydrierung von Carbonylverbindungen, (u.a. Aldehyde und Hydroxyaldehyde wie Hydroxypivalinaldehyd,) insbesondere von Carbonsäuren und deren Derivaten, wobei als Katalysator ein CuO/Al₂O₃/La₂O₃-Metalloxid-Cu-Katalysator eingesetzt wird. Die Hydrierung wird im Allgemeinen bei einer Temperatur von 50 bis 350°C und bei einem Druck von 3 bis 350 bar durchgeführt; in den Beispielen wurde ein Druck von 200 bar bei Temperaturen von 210°C bzw. 190°C gewählt. Es werden dabei hohe Umsätze und Selektivitäten erzielt. Die Bildung von Nebenprodukten wird nicht erwähnt.

CN 103 449 970 A offenbart die Hydrierung von Hydroxypivalaldehyd, welcher durch die Kondensation von Isobutyraldehyd mit Formaldehyd in Gegenwart eines tertiären Amins erhalten wurde, bei einem pH-Wert von 7,5 bis 10, einer Temperatur von 110 bis 140°C, einem Druck von 2 bis 5 MPa abs, in Gegenwart Kupfer und Lanthan enthaltenden SiO₂-Trägerkatalysators.

WO 2011/061185 offenbart die Verwendung eines geträgerten Hydrierkatalysators, der Kupfer als Hydriermetall und Al₂O₃ und Lanthanoxid als Trägermaterial enthält, der zur Hydrierung von Carbonylverbindungen eingesetzt wird. Vor seinem Einsatz in der Hydrierung wird der Katalysator basisch bei einem pH > 10 z.B. mit Natronlaugelösung vorbehandelt. Die Hydrierung erfolgt im Allgemeinen bei einer Temperatur im Bereich von 50 bis 350°C und einem Druck von 3 bis 350 bar. Es werden Beispiele mit Hydrierungen bei 200°C bzw. 100°C und Drücken von 200 bzw. 90 bar gezeigt.

Dieses Verfahren hat, bedingt durch die basische Vorbehandlung des Katalysators, den Nachteil, dass sich bei der Hydrierung von Dimethylolbutyraldehyd zu Trimethylolpropan die Selektivität um 1-2 % verringert. Auf die Bildung von Nebenprodukten wird dabei nicht näher eingegangen.

Bedingt durch Formaldehyd als Reaktionspartner bei der Aldolisierung, enthalten die gebildeten Produkte - neben den gewünschten Carbonylverbindungen - Formiate, deren Vorhandensein auf bereits im eingesetzten Formaldehyd enthaltene Ameisensäure und/oder durch Cannizzaro-Reaktionen während der Umsetzung mit Formaldehyd basiert.

Diese Formiate können in den Folgestufen stören, beispielsweise durch CO-Bildung während einer Hydrierung und/oder durch Veresterung des Produktes, was zu Ausbeuteverlusten bzw. Reinheitsproblemen im Endprodukt führen kann. Ferner können die Formiate z.B. bei der destillativen Aufarbeitung Ameisensäure freisetzen, was dann zu unerwünschten Korrosionen führen kann und den Einsatz von teuren Werkstoffen erfordert.

Aufgabe der vorliegenden Erfindung ist es daher, ein Verfahren bereitzustellen, durch das es gelingt, in Stoffgemischen enthaltene Formiate ganz oder zumindest teilweise abzubauen. Eine weitere Aufgabe der Erfindung ist es, dass bei dem Abbau der Formiate möglichst kein Kohlenmonoxid (CO) gebildet wird.

Gegenstand der Erfindung ist daher ein Verfahren zum Abbau von Formiaten in formiathaltigen Stoffgemischen, welches dadurch gekennzeichnet ist, dass formiathaltige Stoffgemische in Gegenwart von mindestens einem heterogenen Katalysator, der Lanthan enthält, bei einer Temperatur von 80 bis 180°C und einem Druck von 0,1 bis 60 bar umgesetzt werden, und die formiathaltigen Stoffgemische einen pH-Wert von 6,5 bis 10 aufweisen, wobei
- der Katalysator ein Lanthanoxid/Kupfer/Kupferoxid/Aluminiumoxid-Katalysator ist, der 4 bis 15 Gew.-% Lanthan, gerechnet als La₂O₃, bezogen auf das Gesamtgewicht des Katalysators, enthält, und
- das formiathaltige Stoffgemisch Carbonylverbindungen enthält, die durch eine Aldolreaktion von Alkanalen mit Formaldehyd gebildet wurden und/oder deren entsprechende Hydrierprodukte, und
- zumindest 50 Gew.-% der Formiate abgebaut werden.

Beispiele von Carbonylverbindungen sind z.B. Methylolalkanale, die durch eine Aldolreaktion von Alkanalen mit Formaldehyd gebildet wurden. Weiterhin können in dem erfindungsgemäßen Verfahren auch deren entsprechende Hydrierprodukte verwendet werden. Unter Hydrierprodukten sind insbesondere solche zu verstehen bei denen die Aldehyd- oder Ketogruppe der Carbonylverbindungen zum Alkohol reduziert wurde.

Das erfindungsgemäße Verfahren eignet sich im Allgemeinen für die Anwendung vor, während oder nach einer Hydrierung der vorgenannten formiathaltigen Stoffgemische. Bevorzugt wird das erfindungsgemäße Verfahren während oder nach, besonders bevorzugt während einer Hydrierung angewandt.

Im Sinne der vorliegenden Erfindung wird unter Formiat Ameisensäure bzw. dessen Salze oder Ester der Ameisensäure mit Alkoholen verstanden. Bevorzugt sind dies Salze, abgeleitet von Ameisensäure und Aminen, bevorzugt von tertiären Aminen, bevorzugt beispielsweise Trimethylamin, Methyldiethylamin, Dimethylethylamin, Triethylamin. Die Alkylkomponente des Amins kann auch längerkettig und/oder cyclisch sein.

Der Formiatgehalt (gemessen bzw. berechnet als Ameisensäure) im erfindungsgemäß eingesetzten Stoffgemisch kann bis zu 20000 Gew.-ppm betragen, bevorzugt von 10 bis 8000 ppm, besonders bevorzugt von 100 bis 3000 ppm.

Unter den erfindungsgemäßen Bedingungen werden die in dem erfindungsgemäß eingesetzten Stoffgemisch enthaltenen Formiate zumindest zu 50 %, bevorzugt zumindest zu 70 %, besonders bevorzugt zumindest zu 80 % abgebaut.

Der pH-Wert des formiathaltigen Stoffgemischs wird in einer wässrigen Lösung bestimmt, die zumindest 50 Gew.-% Wasser enthält. Dies bedeutet, dass formiathaltige Stoffgemische, die weniger als 50 Gew.-% Wasser enthalten, entsprechend mit Wasser auf 50 % Wassergehalt verdünnt werden. Sollte sich das Gemisch nicht vollständig mischen, so wird der pH aus der wässrigen Phase bestimmt. Bei formiathaltigen Stoffgemischen, die einen Wassergehalt > 50% aufweisen, wird der pH aus eben diesem Gemisch bestimmt.

Erfindungsgemäß weist die wässrige Lösung des formiathaltigen Stoffgemischs, die sogenannte Feedlösung, einen pH-Wert im Bereich von 6,5 bis 10, bevorzugt von 7 bis 9,5, besonders bevorzugt von 7,5 bis 9 auf. Die Bestimmung des pHs erfolgt dabei in der Feedlösung vor Erreichen des Katalysators bzw. vor Kontakt der Feedlösung mit dem Katalysator. Sinkt der pH unter 6,5, so ist die Standzeit des Katalysators eingeschränkt, bei pH-Werten von > 10 finden unerwünschte Nebenreaktionen statt.

Die Einstellung des pH-Werts geschieht bevorzugt durch Dosierung von Base. Hier ist wiederum ein tertiäres Amin bevorzugt. Sofern die Bereitstellung des erfindungsgemäß eingesetzten formiathaltigen Stoffgemischs, sogenannter Feedstrom, durch eine Aldolreaktion in Gegenwart eines tert. Amins erfolgte, so wird zur pH-Wert-Einstellung besonders bevorzugt das gleiche Amin verwendet.

Erfindungsgemäß wird ein Lanthanoxid/Kupfer/Kupferoxid/Aluminiumoxid-Katalysator eingesetzt, in dem Lanthan beispielsweise als La₂O₃ oder in Form einer Spinellstruktur vorliegt. Ganz besonders bevorzugt liegen mindestens 30 Gew.-%, bezogen auf die Gesamtmenge des in dem Katalysator enthaltenen Lanthans als La₂O₃ oder in einer Spinellstruktur vor.

Der Gewichtsanteil des Lanthans (gerechnet als La₂O₃), bezogen auf das Gesamtgewicht des Katalysators, liegt im Bereich von 4 bis 15%.

Der Lanthan-haltige Katalysatoren enthält zudem Kupfer. Das Kupfer kann als Kupferoxid (gerechnet als CuO) in einer Menge von 10 bis 90 Gew.-%, bevorzugt 20 bis 75 Gew.-%, besonders bevorzugt 30 bis 70 Gew.-%, ganz besonders bevorzugt 40 bis 65 Gew.-%, bezogen auf das Gesamtgewicht des Katalysators, enthalten sein. Die angegebenen CuO-Mengen beziehen sich auf den Gehalt vor der Aktivierung des Katalysators mit Wasserstoff. Das Kupferoxid kann dabei zumindest zu 99 Gew.-% durch Fällung eines löslichen Precursors wie z.B. Cu(NO₃)₂ eingebracht werden und zumindest 1 Gew.-%, bevorzugt zumindest 2 Gew.-%, besonders bevorzugt zumindest 3 Gew.-%, ganz besonders bevorzugt mindestens 5 Gew.-% des CuO kann als pulverförmiges Kupfer oder als Kupferblättchen, bezogen auf das Gesamtgewicht des Katalysators vorliegen. Dabei wird das elementar eingebrachte Kupfer vor Verwendung des Katalysators durch Calzination an Luft zu Kupferoxid umgewandelt.

Der erfindungsgemäß eingesetzte Katalysator kann neben Lanthanoxid, Kupfer, Kupferoxid und Aluminiumoxid auch noch weitere Materialen enthalten, wie z.B. pulverförmigen Zement, Aktivkohle oder oxidische Materialen abgeleitet von Si, Al, Zr, Ti oder Gemische daraus. Diese Materialien können in einer Menge von 0,5 - 80 Gew.-%, jeweils bezogen auf das Gesamtgewicht des Katalysators, in dem erfindungsgemäßen Katalysator enthalten sein. Bevorzugt sind Kohlenstoff und SiO₂.

Als Zement wird vorzugsweise ein Tonerdezement eingesetzt. Besonders bevorzugt besteht der Tonerdezement im Wesentlichen aus Aluminiumoxid und Calciumoxid. Ferner kann ein Zement auf Basis Magnesiumoxid/Aluminiumoxid und Calciumoxid/Aluminiumoxid/ Eisenoxid verwendet werden.

Beispiele für besonders bevorzugte für das erfindungsgemäße Verfahren geeignete Lanthan-haltige Katalysatoren und deren Herstellung finden sich in WO 2007/006719 (Seiten 5-8, 13-14).

Erfindungsgemäß ganz besonders bevorzugt sind solche Lanthan-haltigen Katalysatoren, welche auch in der Lage sind, Carbonyl-haltige Verbindungen zu den entsprechenden Alkoholen zu hydrieren. Beispiele für dazu geeignete Katalysatoren werden ebenfalls in WO 2007/006719 (Seiten 5-8, 13-14) beschrieben.

Besonders bevorzugt sind solche Katalysatoren enthaltend oder bestehend aus:
4 bis 15 Gew.-% Lanthanoxid,
40 bis 65 Gew.-% Kupferoxid,
15 bis 30 Gew-% Aluminiumoxid und
5 bis 20 Gew.-% Kupfer
besteht, wobei die Summe der genannten Materialien 100 Gew.-% egibt.

Dabei beziehen sich die vorgenannten Zusammensetzungen der Katalysatoren auf deren Zusammensetzung vor der Calzination mit Luft.

Unter den vorgenannten Katalysatoren insbesondere bevorzugt ist ein Katalysator enthaltend oder bestehend aus (vor der Calzination mit Luft):
58 Gew.-% CuO,
22 Gew.-% Al₂O₃,
5 Gew.-% La₂O₃,
15 Gew.-% Cu.

Der Katalysator wird im Allgemeinen bei Temperaturen von 50 bis 150°C, vorzugsweise bei 120°C getrocknet und im Anschluss gegebenenfalls vorzugsweise 2 Stunden bei im Allgemeinen 200 bis 600°C, insbesondere bei 300 bis 500°C, bevorzugt mit Luft, calciniert. Der vorstehend beschriebene getrocknete und calcinierte Katalysator kann vor seinem Einsatz in dem erfindungsgemäßen Verfahren einer Behandlung mit siedendem Wasser und/oder Dampf unterzogen werden, wodurch eine hohe Stabilität des Formkörpers, der als Katalysator eingesetzt wird, erreicht wird und gleichzeitig die Hydrieraktivität und Selektivität des Katalysators gesteigert wird. Die Wasserbehandlung und/oder Dampfbehandlung des Katalysators kann wie in WO 2007/006719 (Seiten 5 und 6) beschrieben erfolgen. Bevorzugt erfolgt die Behandlung mit siedendem Wasser und/oder Dampf, besonders bevorzugt mit siedendem Wasser.

Nach der Wasser- und/oder Dampfbehandlung wird der Katalysator üblicherweise nochmals bei Temperaturen von im Allgemeinen 50 bis 300°C getrocknet, und gegebenenfalls calciniert.

Die zuvor beschriebene Wasser- oder Dampfbehandlung des Katalysators kann vor während oder nach der nachfolgend beschriebenen Aktivierung des Katalysators erfolgen.

Eine Nachbehandlung des Katalysators mit einer Basenlösung mit einem pH-Wert > 10, wie in WO 2011/061185 beschrieben, ist für das erfindungsgemäße Verfahren ungeeignet und daher explizit vom Schutzumfang ausgenommen.

Der erfindungsgemäß eingesetzte Katalysator wird vor seinem Einsatz mit reduzierenden Gasen wie Wasserstoff oder wasserstoff-haltigen Gemischen, bevorzugt mit Wasserstoff, bei Normaldruck im Bereich von 20 - 250°C, besonders bevorzugt zwischen 100 und 230°C aktiviert.

Die Aktivierung des Katalysators kann in dem Reaktor durchgeführt werden, in dem auch das erfindungsgemäße Verfahren durchgeführt wird. Es ist ebenso möglich, den Katalysator vor Einbau in den Reaktor anderenorts zu aktivieren. Dabei ist es bevorzugt, diesen Katalysator nach der Aktivierung entweder mit einem inerten, flüssigen Stoff wie z.B. Wasser oder Gemischen aus Wasser und Alkoholen wie Neopentylglykol oder iso-Butanol zu überschichten oder oberflächlich mit z.B. Luft zu passivieren. In beiden Fällen kann dieser Katalysator nach Einbau in den Reaktor ohne weitere Behandlung zur Aktivierung verwendet werden.

Das erfindungsgemäße Verfahren findet bevorzugt bei einer Temperatur im Bereich von 90 bis 150°C, besonders bevorzugt im Bereich von 90 bis 130°C statt. Der Druck liegt bevorzugt im Bereich von 0,5 bis 50 bar, besonders bevorzugt im Bereich von 0,8 bis 45 bar.

Gegebenenfalls kann das erfindungsgemäße Verfahren zum Abbau von Formiaten auch in Gegenwart von Wasserstoff erfolgen. Falls während der katalytischen Umsetzung des formiathaltigen Stoffgemischs zusätzlich hydriert werden soll, wird das erfindungsgemäße Verfahren im Allgemeinen bei einem Druck, der im Wesentlichen durch Wasserstoff bestimmt wird, im Bereich von 3 bis 60 bar, bevorzugt im Bereich von 5 bis 50 bar, besonders bevorzugt im Bereich von 8 bis 45 bar durchgeführt. Bei den vorgenannten Drücken handelt es sich um absolute Drücke.

Bei dem erfindungsgemäßen Verfahren entsteht beim Abbau der Formiate kein CO, sondern CO₂ und Wasserstoff. Dieser Wasserstoff kann zur Hydrierung von Carbonylgruppen verwendet werden.

Ein Vorteil des erfindungsgemäßen Verfahrens ist somit, dass keine Katalysatordesaktivierung bzw. Vergiftung mit CO stattfindet.

Wird der Lanthan-haltige Katalysator nicht nur für den Formiat-Abbau, sondern auch für die Carbonylhydrierung erfindungsgemäß eingesetzt wird, ist es überraschend, dass gegenüber einem nicht Lanthan-haltigen Katalysator, eine höhere Aktivität und Lebensdauer resultiert. Die erfindungsgemäß eingesetzten Lanthan-haltigen Katalysatoren zeichnen sich also nicht nur dadurch aus, dass sie Formiate abbauen können, sondern sie sind auch aktiver und robuster als die entsprechenden Katalysatoren ohne Lanthan, was eine längere Lebensdauer bewirkt.

Bei den erfindungsgemäß eingesetzten Formiat-haltigen Stoffgemischen handelt es sich insbesondere um den Reaktionsaustrag der Aldolreaktion eines Aldehyds mit 2 bis 24 C-Atomen mit Formaldehyd wie beispielsweise in WO 98/28253 A1 sowie ferner in WO 2004/092097 A1 und speziell für Isobutyraldehyd in WO 2011/141470 A1 beschrieben. Der in der Aldolreaktion eingesetzte Aldehyd wird dabei im Allgemeinen mit der 1 bis 8-fachen Menge Formaldehyd in Gegenwart eines tertiären Amins zu einem Methylolalkanal umgesetzt. Die in dem Reaktionsaustrag enthaltenen Produkte der Aldolreaktion enthalten mindestens eine Methylolgruppe. Bevorzugt handelt es sich erfindungsgemäß bei dem Methylolalkanal um Hydroxypivalinaldehyd oder 2,2-Bis-hydroxymethylbutanal. Der das Methylolalkanal-enthaltende Reaktionsaustrag enthält als Nebenprodukt Formiate.

Das Formiat-haltige Stoffgemisch, insbesondere der zuvor beschriebene Methylolalkanal enthaltende Reaktionsaustrag, wird dann zum Abbau der gebildeten Formiate dem erfindungsgemäßen Verfahren zugeführt.

Gemäß einer bevorzugten Ausführungsform des erfindungsgemäßen Verfahrens kann gleichzeitig das Methylolalkanal hydriert werden.

Bevorzugte hydrierte Methylolalkanale weisen mindestens 2 Methylolgruppen auf. Von besonderer Bedeutung sind erfindungsgemäß technisch verwendete hydrierte Methylolalkanale wie beispielsweise Neopentylglykol (NPG) oder Trimethylolpropan (TMP).

Bei dem erfindungsgemäßen Verfahren wird im Allgemeinen eine Katalysatorbelastung (kg Feed/Liter Katalysator pro Stunde) von 0,01 bis 100, bevorzugt zwischen 0,05 und 50, besonders bevorzugt zwischen 0,1 und 30 eingestellt. Unter dem Feed ist dabei im Allgemeinen ein Gemisch aus Alkanal, Formiat, gegebenenfalls Wasser und gegebenenfalls Alkanol zu verstehen, das einen pH von 6,5 bis 10 aufweist.

Die Querschnittsbelastung (m³ Feed/m² Fläche pro Stunde) beim kontinuierlichen Durchführen des erfindungsgemäßen Verfahrens liegt zwischen 0,1 und 300.

Sofern das erfindungsgemäße Verfahren ohne zusätzliche Hydrierung durchgeführt wird, liegen die Querschnittsbelastungen bevorzugt im unteren Bereich, d.h. zwischen 0,1 und 50, bevorzugt zwischen 0,5 und 30. Wird das erfindungsgemäße Verfahren bei zusätzlicher Hydrierung durchgeführt, liegen die Querschnittsbelastungen bevorzugt zwischen 10 und 300, besonders bevorzugt zwischen 10 und 100, da bei stark exothermen Reaktionen, zumindest in einem ersten Reaktor, technisch oft mit Produktrückführung zur Wärmeabführung gearbeitet wird.

Im Allgemeinen ist ein Teil des Feeds Wasser, beispielsweise im Gew.-%-Bereich von 0,5 bis 80 %, bevorzugt zwischen 1 und 60 %, besonders bevorzugt zwischen 5 und 50 %.

Als Reaktoren können alle Reaktoren eingesetzt werden, die zum Umgang mit festen Katalysatoren prinzipiell geeignet sind. Beispiele sind diskontinuierlich oder kontinuierlich betriebene Reaktoren, in dem der Katalysator suspendiert vorliegt. Bevorzugt sind allerdings kontinuierlich durchströmte Reaktoren, v.a. wenn Mengen oberhalb von 50 to pro Jahr hergestellt werden sollen, bei denen der Katalysator fest angeordnet ist. Dabei können alle technisch einsetzbaren Geometrien eines Katalysators eingesetzt werden, beispielsweise Tabletten, Stränge, Triloben, Hohltabletten usw. Der bevorzugte Mindestdurchmesser dieser Formkörper beträgt 0,5 mm, besonders bevorzugt 1 mm.

Es kann ein Reaktor verwendet werden, es sind jedoch auch mehrere möglich, entweder parallel oder bevorzugt hintereinander angeordnet.

Sofern zusätzlich zum erfindungsgemäßen Formiatabbau auch hydriert werden soll, ist es bevorzugt, einen ersten Reaktor mit äußerem Flüssigumlauf mit außenliegenden Wärmetauscher zu benutzen, gefolgt von einem zweiten Reaktor, der im geraden Durchgang getrieben wird. Dabei ist es für den Formiatabbau prinzipiell unerheblich, ob die Reaktoren in Sumpf- oder Rieselfahrweise betrieben werden, zur Erzielung eines besseren Hydrierumsatzes ist allerdings die Rieselfahrweise bevorzugt.

Das bei der Reaktion entstehende Gas wird über das Abgas ausgeschleust. Wird die Reaktion unter Druck betrieben, so entweicht das meiste Gas (> 80 %) beispielsweise in einem sog. Druckabscheider, in dem Flüssigkeit und Gas voneinander getrennt werden und das Gas über eine Druckhaltung entspannt wird. Gelöstes Restgas wird dann meist bei Umgebungsdruck bzw. auch in der nachfolgenden Destillation entfernt.

Weiterer Gegenstand der Erfindung ist die Verwendung mindestens eines heterogenen Lanthan-haltigen Katalysators zum Abbau von Formiaten in formiathaltigen Stoffgemischen, die einen pH-Wert von 6,5 bis 10 aufweist, bei einer Temperatur von 80 bis 180°C und einem Druck von 0,1 bis 60 bar. Der Katalysator, die Formiate, die formiathaltigen Stoffgemische, der Druck und die Temperatur sind dabei wie oben zuvor beschrieben.

In den nachfolgenden Beispielen ist das erfindungsgemäße Verfahren näher erläutert, soll dadurch aber nicht eingeschränkt werden.

### Beispiele

Der Formiatgehalt wurde jeweils durch lonenaustauschchromatographie (IC) bestimmt. Der Cu/Al₂O₃-Katalysator wurde analog WO95/32171 A1, Beispiel E hergestellt; der Lanthan-haltige Katalysator nach WO 2007/006719 A1, Beispiel 2.

Die Katalysatoren wurden vor ihrem Einsatz aktiviert. Dabei wurde unter Stickstoff bei Normaldruck auf 180°C aufgeheizt und anschließend dem Stickstoffstrom 10 Volumen-% Wasserstoff zugemischt. Nach 2 h wurde der Gehalt an Wasserstoff schrittweise pro Stunde um 20 % auf 100 % erhöht.

Die Durchführung des Beispiels 1 sowie des Vergleichsbeispiels war wie folgt:
In einem Rohreaktor (1) (10 m Länge) wurde der Katalysator (87 g, 3 x 3 mm Tabletten) wie oben beschrieben aktiviert und anschließend in Rieselfahrweise bei 40 bar und bei nach unten zunehmenden Temperaturen von 98 bis 105°C betrieben. Der Wasserstofffluss betrug 10 Normliter (NL)/Stunde. Der Austrag wurde gesammelt und nach Analyse in einem weiteren Reaktor (2) am jeweils gleichen, aktivierten Katalysator (87 g) wie im ersten Reaktor bei 103°C und 40 bar nachhydriert.

Der Formiatgehalt im Feed lag bei ca. 1500 Gew.-ppm.

Zusammensetzung Feed (Gew.-%): NPG ca. 65 %, Hydroxypivalinaldehyd ca. 5 %, Wasser ca. 25 %, pH 8,1. Andere Komponenten wie Isobutyraldehyd, Trimethylamin, Methanol, Formaldehyd, Isobutanol liegen in Summe unter 5 %.

Die Ergebnisse sind in Tabelle 1 zusammengefasst.

**Tab. 1**

| | Katalysator | Belastung kg Feed/Liter Kat x h | Querschnittsbelastung m³ Feed/m² x h | Betriebszeit | HPA-Umsatz | Formiatgehalt Gew.-ppm |
|---|---|---|---|---|---|---|
| **Beispiel 1** | Analog WO2007/006719 | | | 1 Monat | | |
| Reaktor (1) | | 4,25 | 19 | | 88% | 1000 |
| Reaktor (2) | | 0,90 | 4 | | 100 % | 200 |
| Reaktor (1) | | 7,0 | 32 | weitere 2 Monate | 77% | 1100 |
| Reaktor (2) | | 1,5 | 6,5 | | 100 % | 500 |
| Reaktor (1) | | 4,25 | 19 | 3 Monate +1 Woche | 87% | 1000 |
| Reaktor (2) | | 0,90 | 4 | | 100 % | 200 |
| **VergleichsBeispiel 1** | Analog WO95/32171 | | | 1 Monat | | |
| Reaktor (1) | | 4,25 | 19 | | 85% | 1500 |
| Reaktor (2) | | 0,90 | 4 | | 100 % | 1300 |
| Reaktor (1) | | 7,0 | 32 | weitere 2 Monate | 66% | 1500 |
| Reaktor (2) | | 1,5 | 6,5 | | 100 % | 1400 |
| Reaktor (1) | | 4,25 | 19 | 3 Monate +1 Woche | 75 % | 1500 |
| Reaktor (2) | | 0,90 | 4 | | 100 % | 1500 |

Man erkennt, dass der nicht Lanthan-haltige Katalysator (Vergleichsbeispiel 1) nach 3 Monaten Betriebszeit deutlich deaktivierte und fast kein Formiat abbaute, wohingegen der in dem erfindungsgemäßen Verfahren eingesetzte Lanthan-haltige Katalysator auch nach 3 Monaten noch über eine ausgezeichnete Aktivität verfügt.

Die gesammelten Hydrierausträge aus Beispiel 1 bzw. des Vergleichsbeispiels mit 200 ppm Formiat bzw. 1300 ppm Formiat wurden in einer Destillation in eine überwiegend wasserhaltige Kopffraktion und eine überwiegende NPG-haltige Sumpffraktion aufgetrennt. In der Wasserfraktion fanden sich bei Destillation der Austräge aus Beispiel 1 0,1 % Formiat in Form von Ameisensäure-Aminsalz bzw. NPG-Formiat. Bei Destillation der Austräge aus Vergleichsbeispiel 1 fanden sich 1 % Formiat in Form von Ameisensäure-Aminsalz bzw. NPG-Formiat.

### Beispiele 2 und 3

In einem Rohreaktor (1) (10 m Länge) wurde der Katalysator (87 g, 3 x 3 mm Tabletten) wie oben beschrieben aktiviert und anschließend in Rieselfahrweise bei gegebenen Druck und Temperaturen gemäß Tabelle 2 betrieben. Der Wasserstofffluss betrug 10 NL/Stunde. Der Austrag wurde gesammelt und nach Analyse in einem weiteren Reaktor (2) am jeweils gleichen, aktivierten Katalysator (87 g) wie im ersten Reaktor nachhydriert.

Der Formiatgehalt im Feed lag bei ca. 1500 Gew.-ppm. Zusammensetzung Feed (Gew.-%): NPG ca. 65 %, Hydroxypivalinaldehyd ca. 5 %, Wasser ca. 25 %, pH 8,1. Andere Komponenten wie Isobutyraldehyd, Trimethylamin, Methanol, Formaldehyd, Isobutanol liegen in Summe unter 5 %.
Die Ergebnisse sind in Tabelle 2 zusammengefasst.

**Tab. 2**

| | Katalysator | Belastung kg Feed/Liter Kat x h | Querschnitts-belastung m³ Feed/m² x h | Druck bar | T °C | HPA-Umsatz | Formiatgehalt ppm |
|---|---|---|---|---|---|---|---|
| **Beispiel 2** | Analog WO2007/006 719 | | | | | | |
| Reaktor (1) | | 4,25 | 19 | 10 | 98-105 | 65 % | 700 |
| Reaktor (2) | | 0,90 | 4 | 10 | 103 | 100 % | 20 |
| **Beispiel 3** | Analog WO WO2007/006 719 | | | | | | |
| Reaktor (1) | | 4,25 | 19 | 40 | 120 | 90 % | 800 |
| Reaktor (2) | | 0,90 | 4 | 40 | 120 | 100 % | 50 |

### Beispiel 4

1 kg der gesammelten Hydrierausträge aus Vergleichsbeispiel 1 mit ca. 1500 ppm Formiat wurden in einem Batchversuch mit 200 ml des aktivierten Lanthan-haltigen Katalysators (nach WO WO 2007/006719 A1, Beispiel 2) bei 90°C umgesetzt. Nach 30 min wurden in dem Hydrieraustrag ca. 250 ppm Formiat in Form von Ameisensäure-Aminsalz bzw. NPG-Formiat gefunden, nach 1 h wurden noch 70 ppm Formiat gefunden.

Die vorgenannten Beispiele zeigen, dass es mit dem erfindungsgemäßen Verfahren gelingt, die Formiate effektiv zu 50% oder mehr abzubauen.

## Patentansprüche

1. Verfahren zum Abbau von Formiaten in formiathaltigen Stoffgemischen, **dadurch gekennzeichnet, dass** formiathaltige Stoffgemische in Gegenwart von mindestens einem heterogenen Katalysator, der Lanthan enthält, bei einer Temperatur von 80 bis 180°C und einem Druck von 0,1 bis 60 bar umgesetzt werden, und die formiathaltigen Stoffgemische einen pH-Wert von 6,5 bis 10 aufweisen,
wobei der Katalysator ein Lanthanoxid/Kupfer/Kupferoxid/Aluminiumoxid-Katalysator ist, der 4 bis 15 Gew.-% Lanthan, gerechnet als La₂O₃, bezogen auf das Gesamtgewicht des Katalysators, enthält, und
das formiathaltige Stoffgemisch Carbonylverbindungen enthält, die durch eine Aldolreaktion von Alkanalen mit Formaldehyd gebildet wurden und/oder deren entsprechende Hydrierprodukte, und
zumindest 50 Gew.-% der Formiate abgebaut werden.

2. Verfahren nach Anspruch 1, **dadurch gekennzeichnet, dass** der Abbau der Formiate in Gegenwart von Wasserstoff erfolgt.

3. Verfahren nach Anspruch 1 oder 2, **dadurch gekennzeichnet, dass** der Abbau der Formiate in Gegenwart eines tertiären Amins erfolgt.

4. Verfahren nach einem der Ansprüche 1 bis 3, **dadurch gekennzeichnet, dass** das formiathaltige Stoffgemisch Verbindungen enthält, die zumindest zwei Methylolgruppen aufweisen.

5. Verfahren nach einem der Ansprüche 1 bis 4, **dadurch gekennzeichnet, dass** das formiathaltige Stoffgemisch Hydroxypivalinaldehyd und Neopentylglykol enthält.

6. Verfahren nach einem der Ansprüche 1 bis 5, **dadurch gekennzeichnet, dass** der Katalysator besteht aus:
4 bis 15 Gew.-% Lanthanoxid,
40 bis 65 Gew.-% Kupferoxid,
15 bis 30 Gew-% Aluminiumoxid, und
5 bis 20 Gew.-% Kupfer,
wobei die Summe der genannten Materialien 100 Gew.-% ergibt.

7. Verwendung mindestens eines heterogenen Lanthan-haltigen Katalysators zum Abbau von Formiaten aus formiathaltigen Stoffgemischen, die einen pH-Wert von 6,5 bis 10 aufweisen, bei einer Temperatur von 80 bis 180°C und einem Druck von 0,1 bis 60 bar, wobei der Katalysator ein Lanthanoxid/Kupfer/Kupferoxid/Aluminiumoxid-Katalysator ist, der 0,5 bis 15 Gew.-% Lanthan, gerechnet als La₂O₃, bezogen auf das Gesamtgewicht des Katalysators, enthält, und
das formiathaltige Stoffgemisch Carbonylverbindungen enthält, die durch eine Aldolreaktion von Alkanalen mit Formaldehyd gebildet wurden und/oder deren entsprechende Hydrierprodukte, und
zumindest 50 Gew.-% der Formiate abgebaut werden.

## Claims

1. A process for decomposing formates in formate-containing compositions of matter, which comprises reacting formate-containing compositions of matter in the presence of at least one heterogeneous catalyst comprising lanthanum and at a temperature of from 80 to 180°C and a pressure of from 0.1 to 60 bar, wherein the formate-containing compositions of matter have a pH of from 6.5 to 10,
where the catalyst is a lanthanum oxide/copper/copper oxide/aluminum oxide catalyst, which comprises 4 to 15 wt% of lanthanum reckoned as La₂O₃ and based on the total weight of the catalyst, and
the formate-containing composition of matter comprises carbonyl compounds formed by an aldol reaction of alkanals with formaldehyde and/or their corresponding hydrogenation products, and
at least 50 wt% of formate is decomposed.

2. The process according to claim 1, wherein the decomposition of the formates is effected in the presence of hydrogen.

3. The process according to claim 1 or 2, wherein the decomposition of the formates is effected in the presence of a tertiary amine.

4. The process according to any one of claims 1 to 3, wherein the formate-containing composition of matter comprises compounds comprising at least two methylol groups.

5. The process according to any one of claims 1 to 4, wherein the formate-containing composition of matter comprises hydroxypivalaldehyde and neopentyl glycol.

6. The process according to any one of claims 1 to 5, wherein the catalyst consists of:
4 to 15 wt% of lanthanum oxide,
40 to 65 wt% of copper oxide,
15 to 30 wt% of aluminum oxide, and
5 to 20 wt% of copper,
wherein said materials sum to 100 wt%.

7. The use of at least one heterogeneous lanthanum-containing catalyst for decomposing formates at a temperature of from 80 to 180°C and a pressure of from 0.1 to 60 bar from formate-containing compositions of matter having a pH of from 6.5 to 10,
where the catalyst is a
lanthanum oxide/copper/copper oxide/aluminum oxide catalyst, which comprises 0.5 to 15 wt% of lanthanum reckoned as La₂O₃ and based on the total weight of the catalyst, and
the formate-containing composition of matter comprises carbonyl compounds formed by an aldol reaction of alkanals with formaldehyde and/or their corresponding hydrogenation products, and
at least 50 wt% of formate is decomposed.

## Revendications

1. Procédé de dégradation de formiates dans des mélanges de matières contenant des formiates, **caractérisé en ce que** des mélanges de matières contenant des formiates sont mis en réaction en présence d'au moins un catalyseur hétérogène, qui contient du lanthane, à une température de 80 à 180 °C et à une pression de 0,1 à 60 bar, et les mélanges de matières contenant des formiates présentent un pH de 6,5 à 10,
le catalyseur étant un catalyseur d'oxyde de lanthane/cuivre/oxyde de cuivre/oxyde d'aluminium, qui contient 4 à 15 % en poids de lanthane, calculé en tant que La₂O₃, par rapport au poids total du catalyseur, et le mélange de matières contenant des formiates contenant des composés de carbonyle, qui ont été formés par une réaction d'aldolisation d'alcanals avec du formaldéhyde, et/ou leurs produits d'hydrogénation correspondants, et
au moins 50 % en poids des formiates étant dégradés.

2. Procédé selon la revendication 1, **caractérisé en ce que** la dégradation des formiates a lieu en présence d'hydrogène.

3. Procédé selon la revendication 1 ou 2, **caractérisé en ce que** la dégradation des formiates a lieu en présence d'une amine tertiaire.

4. Procédé selon l'une quelconque des revendications 1 à 3, **caractérisé en ce que** le mélange de matières contenant des formiates contient des composés qui comprennent au moins deux groupes méthylol.

5. Procédé selon l'une quelconque des revendications 1 à 4, **caractérisé en ce que** le mélange de matières contenant des formiates contient de l'hydroxypivalinaldéhyde et du néopentylglycol.

6. Procédé selon l'une quelconque des revendications 1 à 5, **caractérisé en ce que** le catalyseur est constitué par :
4 à 15 % en poids d'oxyde de lanthane,
40 à 65 % en poids d'oxyde de cuivre,
15 à 30 % en poids d'oxyde d'aluminium et
5 à 20 % en poids de cuivre,
la somme des matériaux mentionnés étant de 100 % en poids.

7. Utilisation d'au moins un catalyseur hétérogène contenant du lanthane pour la dégradation de formiates dans des mélanges de matières contenant des formiates, qui présentent un pH de 6,5 à 10, à une température de 80 à 180 °C et à une pression de 0,1 à 60 bar, le catalyseur étant un catalyseur d'oxyde de lanthane/cuivre/oxyde de cuivre/oxyde d'aluminium, qui contient 0,5 à 15 % en poids de lanthane, calculé en tant que La₂O₃, par rapport au poids total du catalyseur, et
le mélange de matières contenant des formiates contenant des composés de carbonyle, qui ont été formés par une réaction d'aldolisation d'alcanals avec du formaldéhyde, et/ou leurs produits d'hydrogénation correspondants, et
au moins 50 % en poids des formiates étant dégradés.
